# EUROPÄISCHE PATENTSCHRIFT

(11) **EP 2 500 090 B1**
(45) Veröffentlichungstag und Bekanntmachung des Hinweises auf die Patenterteilung: **13.07.2016**
(21) Anmeldenummer: 11158474.4
(22) Anmeldetag: 16.03.2011
(51) Int. Cl.: B01J 21/12, B01J 35/00, B01J 35/10, C01B 33/18, C07C 1/20

(54) **Silicium-Aluminium-Mischoxidpulver**
Silicon-aluminium mixed oxide powder
Poudre d'oxyde de mélange silicium-aluminium

(43) Veröffentlichungstag der Anmeldung: 19.09.2012
(73) Patentinhaber: Evonik Degussa GmbH, 45128 Essen (DE)
(72) Erfinder: Schulze, Christian Isfort, 63694 Limeshain (DE); Zanthoff, Horst-Werner, 45481 Mühlheim a.d. Ruhr (DE); Quandt, Thomas, 45772 Marl (DE); Böing, Christian, 50679 Köln (DE)

(56) Entgegenhaltungen:
- EP-A1- 0 995 718
- EP-A1- 1 266 864
- US-A- 4 254 290

## Beschreibung

Die Erfindung betrifft ein Silicium-Aluminium-Mischoxidpulver, dessen Herstellung und Verwendung.

Isoolefine, wie Isobuten, sind wichtige Zwischenprodukte für die Herstellung einer Vielzahl organischer Verbindungen. In technischen Strömen liegen Isoolefine meist zusammen mit anderen Olefinen und gesättigten Kohlenwasserstoffen mit gleicher Kohlenstoffatomzahl vor. Aus diesen Gemischen sind die Isoolefine allein mit physikalischen Trennmethoden wirtschaftlich nicht abtrennbar.

Üblicherweise wird Isobuten aus C₄-Schnitten, beispielsweise der C₄-Fraktion eines Steamcrackers, abgetrennt, indem man nach Entfernung des größten Teils der mehrfach ungesättigten Kohlenwasserstoffe, hauptsächlich des Butadiens, das verbleibende Gemisch zu linearen Butenen umsetzt. Bei der Verwendung von Methanol entsteht aus Isobuten Methyl-tert.-butylether (MTBE), bei der Verwendung von Ethanol Ethyl-tert.-butylether (ETBE) und bei Einsatz von Wasser tert.-Butanol (TBA). Nach ihrer Abtrennung können diese Derivate in Umkehrung ihrer Bildung zu Isobuten gespalten werden. Die Spaltung von Alkyl-tert.-butylethern (ATBE) zu den entsprechenden Isoolefinen und Alkoholen sowie die Spaltung von tertiären Alkoholen zu den entsprechenden Isoolefinen und Wasser kann in Gegenwart von Katalysatoren durchgeführt werden.

Es sind eine Vielzahl von Katalysatoren zur Spaltung von Alkyl-tert.-alkylethern (ATAE), insbesondere MTBE, und tertiären Alkoholen zu den entsprechenden Isoolefinen sowie Alkohol oder Wasser beschrieben. In US 4,254,290 wird ein 3:1 SiO₂/Al₂O₃-Mischoxid als Katalysator eingesetzt.

EP-A-45159 offenbart ein Silicium-Aluminium-Mischoxidpulver zur Spaltung von Alkyl-tert.-alkylethern mit einem Anteil von 2-98 Gew.-% Aluminiumoxid, welches durch Vermahlen und calcinieren von gröberem Material erhalten wird, DE-A-2924869 beschreibt einen Katalysator für die Spaltung von Alkyl-tert.-alkylethern auf Basis eines Silicium-Aluminium-Mischoxides mit 0,06 bis 0,25 Gew.-% Aluminiumoxid. Das Mischoxid wird durch gemeinsame Fällung von Tetraethylorthosilikat und Aluminiumnitratnonahydrat erhalten.

In EP-A-589557 wird ein Silicium-Aluminium-Mischoxid, welches als weitere Komponente ein Alkalioxid oder Erdalkalioxid enthält offenbart. Es wird hergestellt, indem ein durch Fällung hergestelltes Silicium-Aluminium-Mischoxid mit einer wässrigen Lösung eines Alkali- oder Erdalkalisalzes imprägniert wird.

EP-A-1894621 offenbart einen Katalysator, der 0,5 bis 20 Massen-% Alkalimetall- und/oder Erdalkalimetalloxid, 4 bis 30 Massen-%, Aluminiumoxid und 60 bis 95 Massen-% Siliciumdioxid aufweist. Er wird erhalten, indem man ein durch Fällung hergestelltes Silicium-Aluminium-Mischoxid mit einer wässrigen Alkali- oder Erdalkalimetallsalzlösung unter sauren Bedingungen behandelt und anschließend calciniert.

In EP-A-23588 wird ein Verfahren zur Herstellung eines Silicium-Aluminium-Mischoxidpulvers mit einer BET-Oberfläche von 50 bis 200 m²/g und einem Siliciumdioxid-Anteil von 0,5 bis 20 Gew.-% offenbart, bei dem man dampfförmiges Aluminiumchlorid zusammen mit Luft in die Mischkammer eines Brenners überführt, dort mit Wasserstoff und Siliciumtetrachlorid mischt und das Stoffgemisch in einer Reaktionskammer verbrennt.

In EP-A-585544 wird ein Verfahren zur Herstellung eines Silicium-Aluminium-Mischoxidpulvers mit einer BET-Oberfläche von 20 bis 200 m²/g und einem Siliciumdioxid-Anteil von 15 bis 35 Gew.-% offenbart, bei dem man dampfförmige Silicium- und Aluminiumhalogenide zusammen mit einem Traggas in einer Mischeinheit mit Luft, Sauerstoff und Wasserstoff homogen mischt, das Gemisch einem Brenner zuführt und innerhalb einer Brennkammer in einer Flamme zur Reaktion bringt.

In EP-A-850876 wird ein Verfahren zur Herstellung von dotierten Siliciumdioxidpulvern offenbart. Dabei wird ein dampfförmiger Siliciumdioxid-Precursor und als Dotierungskomponente ein Aerosol einer Metallsalz-Lösung in einer Flamme zur Reaktion gebracht. Durch die feine Verteilung der Dotierungskomponente im Aerosol liegt das Dotierungsmedium während der Genese des Oxids fein verteilt in der Gasphase vor, so dass ein homogener Einbau der Dotierungskomponente in das Oxid erfolgt.

In EP-A-995718 wird dieses Verfahren zur Herstellung eines mit Aluminiumoxid dotierten Siliciumdioxidpulvers eingesetzt. Das so hergestellte Pulver soll als Katalysator einsetzbar sein. Es wurde jedoch gefunden, dass damit die Spaltung von Alkyl-tert.-butylethern (ATBE) zu den entsprechenden Isoolefinen und Alkoholen sowie die Spaltung von tertiären Alkoholen zu den entsprechenden Isoolefinen und Wasser nicht befriedigend ist.

Die bekannten Katalysatoren weisen bei der Spaltung von ATBE oder tertiären Alkoholen in Isoolefin sowie Alkohol beziehungsweise Wasser wenigstens einen der folgenden Nachteile auf:
i. Bildung unerwünschter Nebenprodukte wie Dimethylether oder Oligomere der Produktolefine
ii. geringe Standzeit des Katalysators
iii. verstärkte Bildung von Nebenprodukten bei Anhebung der Reaktionstemperatur zum Ausgleich eines Aktivitätsverlustes
iv. aufwändige und damit kostenintensive Herstellung des Katalysators.

Es bestand daher die technische Aufgabe, einen Katalysator, mit dem die vorgenannten Nachteile minimiert oder ganz vermieden werden können, bereitzustellen.

Überraschenderweise wurde nun gefunden, dass die technische Aufgabe gelöst wird durch ein Silicium-Aluminium-Mischoxidpulver, welches überwiegend oder vollständig in Form von aggregierten Primärpartikeln vorliegt und bei dem
a. das Gewichtsverhältnis von (Al₂O₃/SiO₂)ₜₜₗ im Gesamtprimärpartikel 0,002 bis 0,05, bevorzugt 0,003 bis 0,015, besonders bevorzugt 0,005 bis 0,01,
b. das Gewichtsverhältnis (Al₂O₃/SiO₂)_{Oberfläche} der Primärpartikel in einer oberflächennahen Schicht mit einer Dicke von ca. 5 nm kleiner als im Gesamtprimärpartikel und
c. die BET-Oberfläche 50 bis 250 m²/g, bevorzugt 100 bis 200 m²/g, ist.

Das erfindungsgemäße Silicium-Aluminium-Mischoxidpulver zeichnet sich unter anderem dadurch aus, dass der Anteil des Aluminiumoxides gegenüber dem Siliciumdioxid sehr gering ist und das Gewichtsverhältnis (Al₂O₃/SiO₂)_{Oberfläche} der Primärpartikel in einer oberflächennahen Schicht kleiner als im Gesamtprimärpartikel ist. Dies bedeutet, dass die Aluminiumoxidkonzentration an der Oberfläche weiter reduziert ist. Der Gesamtprimärpartikel schließt den Anteil an Siliciumdioxid und Aluminiumoxid in der oberflächennahen Schicht mit ein. Bevorzugt kann ein erfindungsgemäßes Silicium-Aluminium-Mischoxidpulver sein, bei dem (Al₂O₃/SiO₂)ₜₜₗ / (Al₂O₃/SiO₂)_{Oberfläche} 1,3 bis 20, bevorzugt 1,4 bis 10 und besonders bevorzugt 1,6 bis 5 ist, wobei "ttl." für den Gesamtprimärpartikel steht.

In einer bevorzugten Ausführungsform der Erfindung weist das Silicium-Aluminium-Mischoxidpulver ein Gewichtsverhältnis von (Al₂O₃/SiO₂)ₜₜₗ von 0,005 bis 0,015, ein Verhältnis (Al₂O₃/SiO₂)ₜₜₗ / (Al₂O₃/SiO₂)_{Oberfläche} von 1,3 bis 20 und eine BET-Oberfläche von 100 bis 200 m²/g auf.

Es ist daher überraschend, dass das erfindungsgemäße Silicium-Aluminium-Mischoxidpulver beispielsweise bei der Gasphasenspaltung von Methyl-tert.-butylether oder von tert.-Butylalkohol gegenüber den Verfahren aus dem Stand der Technik sowohl eine vergleichbare katalytische Aktivität und eine geringere Bildung unerwünschter Nebenprodukte zeigt.

Unter Mischoxidpulver soll eine innige Mischung der Mischoxidkomponenten Aluminiumoxid und Siliciumdioxid auf atomarer Ebene verstanden werden, bei der die Primärpartikel auch Si-O-Al-Bindungen aufweisen. Die Oberflächen dieser Primärpartikel sind weitestgehend oder vollständig frei von Poren. Bevorzugt können solche erfindungsgemäßen Silicium-Aluminium-Mischoxidpulver sein, die durch Flammenhydrolyse und/oder Flammenoxidation von Silicium- und Aluminiumverbindungen in einer Flamme, erzeugt durch die Reaktion von Wasserstoff und Sauerstoff, erhalten werden. Diese Pulver werden als "pyrogen" oder "fumed" beschrieben. Bei der Reaktion werden zunächst hochdisperse Primärpartikel gebildet, die im weiteren Reaktionsverlauf zu Aggregaten zusammenwachsen und diese weiter zu Agglomeraten zusammenlagern können.

Das Gewichtsverhältnis auf der Oberfläche kann zum Beispiel durch röntgeninduzierte Photoelektronen-Spektroskopie (XPS-Analyse) des Pulvers bestimmt werden. Zusätzliche Information über die Oberflächenzusammensetzung kann durch energiedispersive Röntgenstrahlung (TEM-EDX-Analyse) von einzelnen Primärpartikeln bestimmt werden.

Das Gewichtsverhältnis im Gesamtprimärpartikel wird durch chemische oder physikalisch-chemische Methoden, z.B. Röntgenfluoreszenzanalyse, des Pulvers bestimmt.

Weiterhin wurde gefunden, dass es vorteilhaft sein kann, wenn das Silicium-Aluminium-Mischoxidpulver eine Dibutylphthalat-Zahl, in g Dibutylphthalat (DBP)/100 g Mischoxid, von 300 bis 350 aufweist. Die DBP-Zahl stellt ein Maß für die Struktur von Aggregaten dar. Niedrige Zahlen korrespondieren mit einer niedrigen Struktur, hohe Zahlen mit einer hohen Struktur. Der bevorzugt Bereich von 300 bis 350 entspricht einer hohen Struktur. Bei der DBP-Absorption wird die Kraftaufnahme, beziehungsweise das Drehmoment (in Nm), der rotierenden Schaufeln des DBP-Meßgerätes bei Zugabe definierter Mengen von DBP, vergleichbar einer Titration, gemessen. Dabei ergibt sich für das erfindungsgemäße Pulver ein scharf ausgeprägtes Maximum mit einem anschließenden Abfall bei einer bestimmten Zugabe von DBP. Die Dibutylphthalatabsorption kann beispielsweise mit einem Gerät RHEOCORD 90 der Fa. Haake, Karlsruhe gemessen werden. Hierzu werden 12 g des Silicium-Aluminium-Mischoxidpulvers auf 0,001 g genau in eine Knetkammer eingefüllt, diese mit einem Deckel verschlossen und Dibutylphthalat über ein Loch im Deckel mit einer vorgegebenen Dosierrate von 0,0667 ml/s eindosiert. Der Kneter wird mit einer Motordrehzahl von 125 Umdrehungen pro Minute betrieben. Nach Erreichen des Drehmomentmaximums wird der Kneter und die DBP-Dosierung automatisch abgeschaltet. Aus der verbrauchten Menge DBP und der eingewogenen Menge der Partikel wird die DBP-Absorption berechnet nach: DBP-Zahl (g/100 g) = (Verbrauch DBP in g / Einwaage Pulver in g) x 100.

Ein weiterer Gegenstand der Erfindung ist ein Verfahren zur Herstellung des erfindungsgemäßen Silicium-Aluminium-Mischoxidpulvers, bei dem man
a) einen Dampf, der eine oder mehrere Siliciumverbindungen ausgewählt aus der Gruppe bestehend aus CH₃SiCl₃, (CH₃)₂SiCl₂, (CH₃)₃SiCl und (n-C₃H₇)SiCl₃ enthält und den Dampf einer hydrolysierbaren und oxidierbaren Aluminiumverbindung getrennt oder gemeinsam, mittels eines Traggases, in eine Mischkammer überführt, wobei das Gewichtsverhältnis von Aluminiumverbindung, gerechnet als Al₂O₃, zu Siliciumverbindung, gerechnet als SiO₂, 0,003 bis 0,05 ist,
b) getrennt hiervon wenigstens ein Brenngas und Luft in diese Mischkammer überführt, wobei die Gesamtmenge an Sauerstoff in der Luft mindestens ausreichend ist zur vollständigen Verbrennung des Brenngases und der Siliciumverbindungen und Aluminiumverbindungen
c) das Gemisch aus dem Dampf der Siliciumverbindungen und der Aluminiumverbindungen, Brenngas und Luft in einem Brenner zündet und die Flamme in eine Reaktionskammer hinein verbrennt,
d) anschließend den Feststoff von gasförmigen Stoffen abtrennt, und nachfolgend den Feststoff mit Wasserdampf behandelt.

Das Verfahren kann auch so ausgeführt werden, dass der Dampf der Siliciumverbindungen bis zu 40 Gew.-% SiCl₄ enthalten kann. Besonders bevorzugt kann ein Gemisch aus 65 bis 80 Gew.-% CH₃SiCl₃ und 20 bis 35 Gew.-% SiCl₄ sein. Als Aluminiumverbindung eignet sich bevorzugt Aluminiumchlorid. Das Brenngas wird bevorzugt aus der Gruppe bestehend aus Wasserstoff, Methan, Ethan, Propan und Gemischen hiervon ausgewählt. Besonders bevorzugt ist Wasserstoff. Die in die Mischkammer eingebrachte Luft reicht wenigstens zur vollständigen Verbrennung des Brenngases und der Siliciumverbindungen und Aluminiumverbindungen aus. In der Regel wird ein Überschuß an Luft eingesetzt. Die Behandlung mit Wasserdampf dient dem Zweck an den Partikeln anhaftende Chloridreste weitestgehend zu entfernen, so dass das Pulver nicht mehr als 1 Gew.-% Chlorid, bevorzugt nicht mehr als 0,2 Gew.-% Chlorid, enthält.

Ein weiterer Gegenstand der Erfindung ist die Verwendung des Silicium-Aluminium-Mischoxidpulvers als Katalysator.

### Beispiele

### Beispiel 1: Herstellung des erfindungsgemäßen Silicium-Aluminium-Mischoxidpulvers

Der Dampf eines Gemisches bestehend aus 45 kg/h CH₃SiCl₃ und 15 kg/h SiCl₄ und der Dampf 0,6 kg/h Aluminiumchlorid werden getrennt voneinander mittels Stickstoff als Traggas in eine Mischkammer überführt. Die Dämpfe werden mit 14,6 Nm³/h Wasserstoff und 129 Nm³/h getrockneter Luft in der Mischkammer eines Brenners gemischt, und über ein Zentralrohr, an dessen Ende das Reaktionsgemisch gezündet wird, einem wassergekühlten Flammrohr zugeführt und dort verbrannt. Das entstandene Pulver wird anschließend in einem Filter abgeschieden und mit Wasserdampf bei 400 - 700°C behandelt. Das Pulver enthält 99 Gew.-% Siliciumdioxid und 1 Gew.-% Aluminiumoxid. Die BET-Oberfläche beträgt 173 m²/g. Die DBP-Zahl beträgt 326 g / 100 g Mischoxid.

Zur Bestimmung des Gewichtsverhältnises (Al₂O₃/SiO₂)_{Oberfläche} der Primärpartikel in einer oberflächennahen Schicht mit einer Dicke von ca. 5 nm wird eine XPS-Analyse angewandt. Sie liefert ein Gewichtsverhältnis (Al₂O₃/SiO₂)_{Oberfläche} von 0,0042. Die Bestimmung des Gewichtsverhältnises (Al₂O₃/SiO₂)ₜₜₗ im Gesamtprimärpartikel erfolgt durch Röntgenfluoreszenzanalyse am Pulver. Sie liefert ein Gewichtsverhältnis (Al₂O₃/SiO₂)ₜₜₗ. von 0,010. Damit ergibt sich ein Wert für (Al₂O₃/SiO₂)ₜₜₗ/ (Al₂O₃/SiO₂)_{Oberfläche} von 2,4.

Beispiel 2 (Vergleichsbeispiel): Als Vergleichsmaterial dient das in EP-A-995718, Beispiel 2 offenbarte Silicium-Aluminium-Mischoxidpulver mit einem Al₂O₃-Anteil von 0,27 Gew.-%. Es wird nach einem in EP-A-850876 offenbarten Verfahren hergestellt, welches es ermöglicht den Aluminiumoxidanteil homogen zu verteilen. Das Pulver weist eine BET-Oberfläche von 204 m²/g.

Beispiel 3 (Vergleichsbeispiel): 24,5 Nm³/h Wasserstoff werden zusammen mit 129 Nm³/h Luft und 80 kg/h SiCl₄-Dampf vermischt, anschließend 8,1 kg/h AlCl₃-Dampf hinzugegeben und das Reaktionsgemisch in einem Flammrohr verbrannt, wobei in dieses Flammrohr zusätzlich 80 Nm³/h Luft eingespeist werden. Nach dem Passieren des Flammrohrs wird das entstehende Pulver in einem Filter von gasförmigen Stoffen abgetrennt und anhaftende Salzsäurereste durch Behandlung mit Wasserdampf entfernt.

Das Pulver weist einen Anteil an Aluminiumoxid von 9,3 Gew.-% auf. Die BET-Oberfläche beträgt 195 m²/g.

Beispiel 4: Gasphasenspaltung von Methyl-tert.-butylether (MTBE) : Die Pulver der Beispiele 1 bis 3 werden im Verhältnis 1:5 mit granuliertem Quartz gemischt. Als Referenzmaterial wird Specialyst 071, Evonik Degussa GmbH, verwendet, welches ein mit 10 Massen-% Magnesium (gerechnet als MgO) dotiertes Alumosilikat mit 21 Massen-% Aluminiumgehalt (gerechnet als Al₂O₃), ist. Die spezifische Katalysatorbelastung (WHSV; Gramm Feed je Gramm Katalysator je Stunde) wird zwischen 5 and 50 h⁻¹ variiert.
Tabelle 1 zeigt die Ergebnisse der MTBE-Spaltung mit dem erfindungsgemäßen Pulver aus Beispiel 1 in Vergleich mit den nicht erfindungsgemäßen Pulvern der Beispiele 2 und 3, sowie dem Referenzmaterial kommerziellen Katalysator Specialyst 071 (ebenfalls als Pulver).

**Tabelle 1: Spaltung von MTBE**

| Pulver | WHSV | MTBE-Umsatz | DME-Selektivität | C₈-Selektivität |
|---|---|---|---|---|
| | h⁻¹ | % | % | % |
| Beispiel 1 | 18 | 85 | 0.63 | 0.06 |
| Beispiel 2 | 25 | 85 | 1.49 | 0.17 |
| Beispiel 3 | 18 | 85 | 1.16 | 0.03 |
| Specialyst 071 | 8 | 85 | 2.29 | 0.10 |

Reaktionsbedingungen: 225°C, 6barü, 0,2g Katalysator; Ergebnisse wurden nach 100h on stream erhalten.

Man kann erkennen, dass das Referenzmaterial mit einer DME-Selektivität von 2.29% den höchsten Wert aller getesteten Pulver aufweist. Die katalytische Aktivität ist ebenfalls relativ gering, da eine niedrige WHSV von 8 h⁻¹ eingestellt werden muss, um den Umsatz von 85% zu erreichen. Die C₈-Selektivität ist ebenfalls mit 0.1% recht hoch.

Im Vergleich dazu zeigt das erfindungsgemäße Pulver aus Beispiel 1 die besten Ergebnisse. Der Umsatz von 85% wird bereits mit einer WHSV von 18 h⁻¹ erreicht. Gleichzeitig sind die Selektivitäten zu DME und C₈ mit Werten von 0.63% bzw. 0.06% sehr niedrig.

Das Pulver aus Beispiel 2 zeigt noch niedrigere Selektivitäten zu den Nebenprodukten, kommt jedoch aufgrund seiner geringen katalytischen Aktivität nur zu einem Umsatz von 35% bei einer WHSV von 8⁻¹.

Das Pulver aus Beispiel 3 zeigt eine schlechtere DME-Selektivität, 1.16%, als das erfindungsgemäße Pulver aus Beispiel 1.

## Patentansprüche

1. Silicium-Aluminium-Mischoxidpulver, welches überwiegend oder vollständig in Form von aggregierten Primärpartikeln vorliegt, **dadurch gekennzeichnet, dass**
a. das Gewichtsverhältnis von (Al₂O₃/SiO₂)ₜₜₗ im Gesamtprimärpartikel 0,003 bis 0,05,
b. das Gewichtsverhältnis (Al₂O₃/SiO₂)_{Oberfläche} der Primärpartikel in einer oberflächennahen Schicht mit einer Dicke von ca. 5 nm kleiner als im Gesamtprimärpartikel und
c. die BET-Oberfläche 50 bis 250 m²/g, ist.

2. Silicium-Aluminium-Mischoxidpulver nach Anspruch 1,
**dadurch gekennzeichnet, dass**
(Al₂O₃/SiO₂)ₜₜₗ / (Al₂O₃/SiO₂)_{Oberfläche} 1,3 bis 20 ist.

3. Silicium-Aluminium-Mischoxidpulver nach den Ansprüchen 1 oder 2,
**dadurch gekennzeichnet, dass**
die Dibutylphthalat-Zahl, in g Dibutylphthalat/100 g Mischoxid, 300 bis 350 ist.

4. Verfahren zur Herstellung des Silicium-Aluminium-Mischoxidpulvers gemäß der Ansprüche 1 bis 3,
**dadurch gekennzeichnet, dass** man
a) einen Dampf, der eine oder mehrere Siliciumverbindungen ausgewählt aus der Gruppe bestehend aus CH₃SiCl₃, (CH₃)₂SiCl₂, (CH₃)₃SiCl und (n-C₃H₇)SiCl₃ enthält und den Dampf einer hydrolysierbaren und/oder oxidierbaren Aluminiumverbindung getrennt oder gemeinsam, mittels eines Traggases, in eine Mischkammer überführt, wobei das Gewichtsverhältnis von Aluminiumverbindung, gerechnet als Al₂O₃, zu Siliciumverbindung, gerechnet als SiO₂, 0,003 bis 0,05 ist,
b) getrennt hiervon wenigstens ein Brenngas und Luft in diese Mischkammer überführt, wobei die Gesamtmenge an Sauerstoff in der Luft mindestens ausreichend ist zur vollständigen Verbrennung des Brenngases und der Siliciumverbindungen und Aluminiumverbindungen
c) das Gemisch aus dem Dampf der Siliciumverbindungen und der Aluminiumverbindungen, Brenngas und Luft in einem Brenner zündet und die Flamme in eine Reaktionskammer hinein verbrennt,
d) anschließend den Feststoff von gasförmigen Stoffen abtrennt, und nachfolgend den Feststoff mit Wasserdampf behandelt.

5. Verfahren nach Anspruch 4,
**dadurch gekennzeichnet, dass**
der Dampf der Siliciumverbindungen bis zu 40 Gew.-% SiCl₄ enthält.

6. Verwendung des Silicium-Aluminium-Mischoxidpulvers gemäß der Ansprüche 1 bis 3 als Katalysator.

## Claims

1. Silicon-aluminum mixed oxide powder which is present predominantly or entirely in the form of aggregated primary particles, **characterized in that**
a. the weight ratio of (Al₂O₃/SiO₂)ₜₜₗ in the total primary particle is from 0.003 to 0.05,
b. the weight ratio (Al₂O₃/SiO₂) surface of the primary particles in a surface layer having a thickness of about 5 nm is less than in the total primary particle and
c.the BET surface area is from 50 to 250 m²/g.

2. Silicon-aluminum mixed oxide powder according to Claim 1, **characterized in that** (Al₂O₃/SiO₂)ₜₜₗ/(Al₂O₃/SiO₂)_{surface} is from 1.3 to 20.

3. Silicon-aluminum mixed oxide powder according to Claim 1 or 2, **characterized in that** the dibutyl phthalate number, in g of dibutyl phthalate/100 g of mixed oxide, is from 300 to 350.

4. Process for preparing the silicon-aluminum mixed oxide powder according to any of Claims 1 to 3, **characterized in that**
a) a vapor containing one or more silicon compounds selected from the group consisting of CH₃SiCl₃, (CH₃)₂SiCl₂, (CH₃)₃SiCl and (n-C₃H₇)SiCl₃ and the vapor of a hydrolysable and/or oxidizable aluminum compound are introduced separately or together by means of a carrier gas into a mixing chamber, where the weight ratio of aluminum compound, calculated as Al₂O₃, to silicon compound, calculated as SiO₂, is from 0.003 to 0.05,
b) separately therefrom, at least one fuel gas and air are introduced into this mixing chamber, where the total amount of oxygen in the air is at least sufficient for complete combustion of the fuel gas and the silicon compounds and aluminum compounds,
c) the mixture of the vapor of the silicon compounds and the aluminum compounds, fuel gas and air is ignited in a burner and the flame burns into a reaction chamber,
d) the solid is subsequently separated from gaseous materials and the solid is subsequently treated with water vapor.

5. Process according to Claim 4, **characterized in that** the vapor of the silicon compounds contains up to 40% by weight of SiCl₄.

6. Use of the silicon-aluminum mixed oxide powder according to any of Claims 1 to 3 as catalyst.

## Revendications

1. Poudre d'oxyde mixte de silicium-aluminium, qui se trouve principalement ou complètement sous forme de particules primaires agglomérées, **caractérisée en ce que**
a. le rapport pondéral (Al₂O₃/SiO₂)ₜₒₜₐₗ dans l'ensemble des particules primaires est de 0,003 à 0,05,
b. le rapport pondéral (Al₂O₃/SiO₂) surface des particules primaires dans une couche, proche de la surface, d'une épaisseur d'environ 5 nm, est inférieur à celui dans l'ensemble des particules primaires et
c. la surface BET est de 50 à 250 m²/g.

2. Poudre d'oxyde mixte de silicium-aluminium selon la revendication 1, **caractérisée en ce que** (Al₂O3/SiO₂)ₜₒₜₐₗ/(Al₂O₃/SiO₂) surface vaut 1,3 à 20.

3. Poudre d'oxyde mixte de silicium-aluminium selon la revendication 1 ou 2, **caractérisée en ce que** l'indice de phtalate de dibutyle, en g de phtalate de dibutyle/100 g d'oxyde mixte, vaut 300 à 350.

4. Procédé pour la préparation de la poudre d'oxyde mixte de silicium-aluminium selon les revendications 1 à 3, **caractérisé en ce que**
a) on transfère une vapeur, qui contient un ou plusieurs composés du silicium, choisis dans le groupe constitué par CH₃SiCl₃, (CH₃)₂SiCl₂, (CH₃)₃SiCl et (n-C₃H₇) SiCl₃, et la vapeur d'un composé d'aluminium hydrolysable et/ou oxydable, séparément ou ensemble, au moyen d'un gaz support, dans une chambre de mélange, le rapport pondéral du composé d'aluminium, calculé sous forme d'Al₂O₃, au composé de silicium, calculé sous forme de SiO₂, valant 0,003 à 0,05,
b) on transfère, séparément de celles-ci, au moins un gaz combustible et de l'air dans cette chambre de mélange, la quantité totale d'oxygène dans l'air étant au moins suffisante pour la combustion complète du gaz combustible et des composés de silicium et des composés d'aluminium,
c) on enflamme le mélange de la vapeur des composés de silicium et des composés d'aluminium, le gaz combustible et l'air dans un brûleur et on brûle la flamme qui entre dans une chambre de réaction,
d) on sépare ensuite le solide des substances gazeuses, puis on traite le solide avec de la vapeur d'eau.

5. Procédé selon la revendication 4, **caractérisé en ce que** la vapeur des composés de silicium contient jusqu'à 40% en poids de SiCl₄.

6. Utilisation de la poudre d'oxyde mixte de silicium-aluminium selon les revendications 1 à 3 comme catalyseur.
